# EUROPEAN PATENT SPECIFICATION

(11) **EP 0 703 730 B1**
(45) Date of publication and mention of the grant of the patent: **12.12.2001**
(21) Application number: 95916672.9
(22) Date of filing: 18.04.1995
(51) Int. Cl.: A23K 1/16, A23K 1/18, A23L 1/035, A61K 7/00, C11B 3/00

(54) **STABLE WATER-IN-OIL EMULSIONS**
STABILE WASSER-IN-OEL EMULSIONEN
EMULSIONS HUILE DANS EAU STABLES

(30) Priority: 18.04.1994 EP 94201067
(43) Date of publication of application: 03.04.1996
(73) Proprietor: DSM N.V., 6411 TE Heerlen (NL)
(72) Inventor: EDENS, Luppo, NL-3062 JL Rotterdam (NL); MEIJER, Dirk, NL-4835 GB Breda (NL); VAN PARIDON, Petrus Andreas, NL-2271 RD Voorburg (NL)
(74) Representative: Visser-Luirink, Gesina, Dr.
(86) International application number: EP9501461
(87) International publication number: WO9528092

(56) References cited:
- EP-A- 0 174 377
- EP-A- 0 247 552
- EP-A- 0 354 356
- EP-A- 0 657 105
- WO-A-92/06599
- WO-A-93/14645
- WO-A-93/16175
- US-A- 3 958 033
- US-A- 3 966 632
- US-A- 4 869 919
- DATABASE WPI Week 7821 Derwent Publications Ltd., London, GB; AN 78-37849A & SU,A,562 281 (MOSCOW SVOBODA COSMETIC) , 12 September 1977
- DATABASE WPI Week 9318 Derwent Publications Ltd., London, GB; AN 93-149429 & JP,A,05 086 368 (MARINO FORUM 21 SH) , 6 April 1993
- JOURNAL OF FISH DISEASES, vol. 12, no. 6, 1989 OXFORD, GB, pages 579-584, A. LILLEHAUG 'Oral immunization of rainbow trout, Salmo gairdneri Richardson, against vibriosis with vaccines protected against digestive degradation'
- DATABASE WPI Week 8124 Derwent Publications Ltd., London, GB; AN 81-43005D & JP,A,56 045 405 (SHISEIDO KK) , 25 April 1981
- DATABASE WPI Week 8916 Derwent Publications Ltd., London, GB; AN 89-118059 & JP,A,01 063 032 (KAO CORP) , 9 March 1989

## Description

### Technical field

The present invention describes stable water-in-oil emulsions. The emulsions are used to add labile compounds to feed, food or cosmetic compositions.

### Background of the invention

Several additives for food, feed and cosmetics are regarded labile, such as colourants, vitamins, vaccines or enzymes.

Additives can be chemically or enzymatically incompatible with other compounds present in a composition. Otherwise, additives can be heat labile and therefore tend to be deactivated during processing steps which occur at an elevated temperature. For example, feed or food is often prepared by using an extrusion process. These extrusion processes are necessary for obtaining food or feed particles in the required shape. Furthermore, there is a growing tendency to include all sorts of additives to a basic feed or food. Feed or food compositions are more often prepared complete with additives which previously used to be added independently.

Several solutions have been suggested to overcome degradation of a labile compound. One solution is the addition of the desired compounds subsequent to a process step at elevated temperature, e.g. after the extrusion process. Different reports have been published concerning formulations of labile compounds and/or for methods to add such compounds to feed or food particles.

DE 2602260 describes the use of a suspension of an enzyme in a liquid or a molten edible fat. After cooling of the mixture the material has to be particularized. After rough destruction the particles are further treated for example by milling. This process has the disadvantage that the milling of powders to guarantee a small particle size is very tedious and leads to undesirable dusts.

International Patent Application WO 93/14645 solves the problem by addition of an oil suspension containing the heat-labile compounds after the extrusion process. The addition of the suspension to the pellets is performed under lowered pressure.

International Patent Application WO 92/06599 describes the use of a water-in-oil emulsion for protecting vaccines. There is no mentioning of the use of a (protein) stabilizing (i.e. a water activity lowering) agent as part of the emulsion. The formulation of labile compounds such as enzymes in such an emulsion would give rise to a rapid loss of enzyme activity. This loss may amount to 50% per month in hot climates which is unacceptable for commercial products. This patent application also describes the preferred application of a lecithin-based emulsifier.

International Patent Application WO 93/16175 describes a stabilized, aqueous enzyme solution comprising urea and/or a polyol as stabilizing agent. Urea and polyols are known agents to stabilize an enzyme in an aqueous environment.

US Patent 3,966,632 discloses a water-in-oil emulsion wherein the water phase contains 10% glucose and the enzymes glucose oxidase and catalase. The glucose-glucose oxidase / catalase system functions as an antioxidant.

In the area of cosmetics there also is a growing tendency to include labile compounds in oil containing end products. Especially compounds aimed at skin protection, cleansing and rejuvenation are of obvious interest. Labile compounds claimed to have beneficial effects in this respect include enzymes, such as proteases.

To guarantee enzyme activity in cosmetic formulations over extended shelf life periods, essentially water-free, hydrophobic compounds such as waxes, plant or mineral oils commonly are used as enzyme formulation agents. Although this approach implies a high storage stability of the enzyme in the cosmetic formulation, a serious disadvantage is that the enzyme can only be activated by exposing the formulations to relatively large quantities of water (during which the initially water-free enzyme, is dissolved). An inherent problem is that enzyme activation is relatively slow and that much of the initial enzyme activity is wasted. This approach is not very convenient in several cosmetic applications.

In an alternative approach as described in J03004791, protein decomposing enzymes to be used in cosmetics are stabilized by adding polysaccharides to aqueous solution of the enzymes. Although this approach minimises the use of water needed to re-activate the enzyme, the approach disregards the need for a lipophilic phase in many cosmetic formulations.

### Summary of the invention

The present invention discloses stable water-in-oil emulsions. Specifically, the present invention discloses a water-in-oil emulsion characterized in that it comprises the following components: water, oil, an enzyme, a water activity-lowering polyol and an emulsifier.

The water-in-oil emulsions of the present invention comprise an enzyme dissolved in the water phase and stabilized by addition of a water activity lowering polyol.

The polyol is added to the water phase of the water-in-oil emulsion in high concentrations, i.e. in amounts of 30-70% (w/w).

A preferred composition comprises a water-in-oil emulsion containing an enzyme in the water phase together with a polyol, and wherein the emulsion is stabilized using an emulsifier which is active at high concentrations of the polyol. The emulsifier which is used preferably is a distilled monoglyceride or polyglycerol polyricineolate.

The presented emulsions are used to protect enzymes before addition to food, feed, cosmetic or oily compositions.

The water-in-oil emulsions of the present invention are stable for prolonged periods of time. They can be used for the preparation of human or animal food compositions. For instance, the water-in-oil emulsions of the present invention are sprayed on feed or food particles. Preferably, the emulsions are sprayed on feed or food particles after dilution of the emulsion in an oil or fat. In addition, the emulsions of the present invention can be used for the preparation of cosmetic compositions.

### Detailed description of the invention

The present invention discloses a water-in-oil emulsion containing in the water phase an enzyne which is labile together with a water activity-lowering polyol, and wherein the emulsion is stabilized using a suitable emulsifier.

The water-in-oil emulsion comprises the following components: water, oil, an enzyme, a water activity-lowering polyol and an emulsifier. The enzymes are present in the water phase. The polyol is added to the water phase to stabilize the desired enzyme.

The enzyme can be chemically or enzymatically incompatible with other compounds. An incompatible compound can be present either in the emulsion or in the composition to which the emulsion is added. In addition, the enzyme can be heat labile.

The present invention discloses stable water-in-oil emulsions comprising more than one enzyme in the water phase. The present invention also discloses stable water-in-oil emulsions comprising an enzyme and a compound incompatible with the enzyme in the water phase. The physical separation of these incompatible compounds is advantageously accomplished by their incorporation in individual water droplets in the oil phase of the emulsion.

Suitable enzymes include proteases, phytases, carbohydrases, lipases, phospholipases and oxidoreductases.

The emulsions of the present invention are especially useful to formulate mutually incompatible enzymes, e.g. enzyme mixtures of which one of the enzymes is a proteolytic enzyme.

Furthermore, the emulsions of the present invention enable an exact dosage of enzymes. First of all because stability of the enzymes is guaranteed, additionally because dosage of a liquid emulsion, in particular of an emulsion diluted in oil, is more precise than dosage of a powder.

The water-in-oil emulsions of the present invention contain a water phase containing the desired enzyme in a highly concentrated form. The desired enzyme can be added to the water phase in an amount of 0.01-30% (w/w) on basis of the dry weight of the active compound.

The enzymes are stabilized by the addition of a water activity lowering polyol. Polyols that are particularly useful are glycerol, sorbitol, sucrose, polypropylene glycol, trehalose, maltodextrins, lactose and glucose.

In addition, the water activity lowering polyol will act to prevent microbial growth in the water phase of the emulsion. If required, the microbial stability of the water phase can be further enhanced by incorporation of generally accepted (foodgrade) antimicrobial agents, such as sorbates or benzoates.

The amount of a water activity lowering polyol to be added to the water phase depends on the amount of the polyol which is required to obtain the desired stabilizing or the desired antimicrobial effect. Polyols are added to the water phase in amounts of 30-70% (w/w).

To obtain stable emulsions, an emulsifier is used. Emulsifiers are surface-active substances which allow one liquid phase to be dispersed in another liquid phase. Emulsifiers possess both hydrophilic and lipophilic groups, the ratio of these groups is known as the HLB value.

In general, fat-soluble, hydrophobic emulsifiers have HLB values in the range of 0-9, while water-soluble substances have HLB values between 11 and 20. Suitable emulsifiers or mixtures of emulsifiers for stabilizing water-in-oil emulsions are claimed to have HLB values in the lower range.

Surprisingly, well known emulsifiers such as Span™ 80 (HLB=4.3), Tween™ 80 (HLB=15) and mixtures thereof do not give rise to stable water-in-oil emulsions in the presence of relatively high amounts of polyols. Lecithin-based emulsifiers, like Emulbesto™ 2000, Emulfluid™ E likewise result in fast phase separation.

The present invention for the first time describes emulsifiers which are active in the presence of relatively high amounts of polyols. Emulsifiers which work in the presence of amounts of polyols as high as 50% have been found.

Useful emulsifiers are monoglycerides (such as Hymono™ 1163 and Hymono™ 7804) and polyglycerol polyricinoleate (Admul™ WOL 1403). Due to the efficacy of the selected emulsifiers in the presence of relatively high amounts of polyols, coalescence of individual water droplets present in the oil phase is low. In this way, the emulsion according to the invention adequately prevents the migration of non-oil soluble compounds between individual water droplets in the emulsion.

Polyglycerol polyricinoleate (Admul™ WOL 1403) was found to be particularly useful in combination with phytase.

Polyglycerol polyricinoleate is liquid at room temperature and does not require heating up prior to or during mixing with oil. The viscosity of the emulsion remains low when using polyglycerol polyricinoleate and almost no droplet flocculation occurs. In addition, the use of polyglycerol polyricinoleate allows for a high concentration of the water phase in the emulsion.

Oils used in the emulsions of the present invention are the oils normally used in food, feed or cosmetic preparations. They include fish oil, soya oil, rapeseed oil, olive oil, cornflower oil, palm oil, avocado oil and different mineral oils.

The invention further discloses a method for the preparation of said water-in-oil emulsions.

The emulsions can be prepared by input of a high amount of energy. For example by severe stirring or by applying high shearing forces. In this way, stable emulsions are obtained, which can be stably stored in this form.

The present invention discloses emulsions which are stable during a long storage time. The emulsions are stable for one month, more preferably for six months, most preferably for more than a year.

The water-in-oil emulsions according to the present invention can be employed as additives for food, feed or cosmetics.

The use of the emulsions of the invention enables food, feed or cosmetic manufacturers to apply enzymes in process steps which would otherwise lead to deactivation of said enzymes. Furthermore, the use of the emulsions of the invention adequately prevents the migration of non-oil soluble compounds from a feed, food or cosmetic composition to the water phase of the emulsion. In this way, the enzyme(s) of interest present in the water phase of the emulsion is/are adequately protected against chemical or enzymatical incompatibilities present in the composition to which the emulsion is added.

The emulsions of the present invention are used in the preparation of human or animal food or feed particles, especially in the preparation of particles for fish or poultry feed. Preferably, the emulsions are added by spraying on food or feed particles. The addition can also be performed under reduced pressure, as described in International Patent Application WO 93/14645.

The emulsions can be used as such to apply to the food or feed particles. Preferably, the emulsions are diluted in an oil or fat before addition to the food or feed particles. An advantage of this process is that the concentrated enzyme can easily, that is with low energy input, be homogeneously mixed with the oil phase. In this way an even distribution of the active compound in the oil is guaranteed. The oil, which then contains the diluted desired enzyme can subsequently be sprayed on the food or feed particle. This process requires a minimal investment in preparing the final mixture to be sprayed on the particles.

In another application the emulsion of the present invention can be used to improve the degumming of soy and rapeseed oil. In this case the emulsion is containing phospholipase A2. Degumming is the removal of lecithin from the indicated oils. The use of phospholipase for degumming has been extensively described in EP 0 513 709. This enzyme improves the water solubility of non-hydratable phospholipids, improving their removal from the oil. The water-in-oil emulsions of the present invention facilitate the addition of the phospholipase to the indicated oils.

### Example 1

### Physical stability of water-in-oil emulsions using different emulsifiers and different enzymes

### 1a. Composition of enzyme solutions

Stabilities of the various water-in-oil emulsions were demonstrated using a food-grade, neutral protease and a feed-grade phytase.

The standard solution of the protease contained 20 g of Protease B500 (Gist-brocades, Holland) dissolved in 80 g of water. After complete dissolution, either sorbitol or glycerol was added and dissolved to reach the designated concentration.

The standard solution of phytase contained either the raw fermentation concentrate (Natuphos® UF concentrate, obtained by ultrafiltration of a germ-free Aspergillus culture filtrate) or the standardized fermentation product Natuphos® 5000 (Gist-brocades, Holland) containing 40% sorbitol. The phytase solutions used in the various experiments contained glycerol or sorbitol in end concentration between 35 to 50%.

### 1b. Emulsions prepared with Span™ 80 / Tween™ 80 mixtures

To 5 g of fish oil (capelin oil as obtained from Skretting, Norway), 0.5 g of either Span™ 80 or Tween™ 80 emulsifier (both from Brocacef, the Netherlands) was added. Additionally, an emulsifier mix was prepared containing different ratios of the above prepared Span™ 80 and Tween™ 80 solutions. After thorough mixing, 0.18 g of one of the above-described stabilized enzyme solutions was added, followed by extensive vortexing.

All emulsions containing either protease or phytase in combination with one of the various Span™ 80 and/or Tween™ 80 emulsifiers showed separation of the oil and aqueous layer within a few hours at room temperature.

### 1c. Emulsions prepared with lecithin-based emulsifiers

Emulbesto™ 2000, Emulfluid™ E and VP627 are all lecithin-based emulsifiers and obtained from Lucas Meyer, Germany. Following the approach outlined under (1b), emulsions were prepared using 0.5 g of each of the above-mentioned lecithin-based emulsifiers. Within a few hours all emulsions prepared with these emulsifier products showed separation of the oil and aqueous layer.

### 1d. Emulsions prepared with monoglyceride emulsifiers

Hymono™ 1163 and Hymono™ 7804 were obtained from Quest, Holland.

Under gentle stirring 24 g of Hymono™ (a monoglyceride; E471) is dissolved in 700 g of fish oil at a temperature of 70°C (Hymono™ 1163) or 50°C (Hymono™ 7804). Subsequently, the solution is cooled down till at least 50°C. Using a high speed homogenator (Ultra Turrax), 200 g of enzyme solution is dispersed in the emulsifier-containing oil phase and the emulsion is cooled down to room temperature. After a period of a few days, the water phase showed very limited sedimentation only. This sedimentation could be minimised by addition of palmitic acid.

### 1e. Emulsions prepared with polyglycerol polyricinoleate

Admul™ WOL 1403 (polyglycerol polyricinoleate) was obtained from Quest, Holland. In combination with labile compounds this emulsifier has several advantages, a.o. the fact that Admul™ WOL 1403 is a liquid at room temperature which allows easy dissolution in the oil phase. This circumvents the need for heating up the oil, so that the handling is easier and the labile compounds can be added to the mixture of oil/emulsifier at room temperature, thereby minimising thermal stress.

To prepare an emulsion with Admul™ WOL 1403, 3 g of Admul™ WOL 1403 was mixed with 87.5 g of fish oil, followed by the addition of 25 g of Natuphos® 5000 (stabilized with 50% sorbitol). The viscosity of the emulsion remained low, the droplet size of the water phase was about 2 µ and no association of the droplets occurred. This indicates the excellent emulsifying property of Admul™ WOL 1403 in this system. The low viscosity allows a much higher concentration of the water phase containing the enzyme in the final emulsion.

By increasing the quantity of Admul™ from 3 g to 9 g, it appeared to be possible to emulsify a mixture of 75 g Natuphos® 5000 Liquid in 87.5 g fish oil. This emulsion is physically stable for a period of 6 months at least. With the phytase enzyme, the Admul™ emulsion is superior over the Hymono™ emulsions in terms of flocculation behaviour and is physically more stable than the Hymono™ emulsions (see Table 1).

**Table 1.**

| Comparison of the physical parameters and properties of the Hymono™ 1163 and Admul™ WOL 1403 emulsions. | | |
|---|---|---|
| PHYSICAL PARAMETERS AND PROPERTIES | HYMONO 1163 | ADMUL WOL |
| temperature to dissolve the emulsifier | 70°C | R.T. |
| temperature during addition of the enzyme | 50°C | R.T. |
| energy to prepare the emulsion | high | low |
| viscosity of the emulsion | medium | low |
| droplet flocculation | medium | low |
| droplet size | 2-10µ | 2µ |
| maximal concentration of water phase in the emulsion | ca 22% (w/w) | ca 44% (w/w) |

### Example 2

### Enzyme activity in a neutral protease water-in-oil emulsion

### Solution A:

Under gentle stirring 5 g of a spray dried Protease B500 preparation is dissolved in 20 g demi water. The protease solution is diluted with glycerol to obtain a final glycerol concentration of 50%.

### Solution B:

Under gentle stirring 6 g of Hymono™ 1163 is dissolved in 175 g of fish oil at a temperature of 70°C. Subsequently, the solution is cooled down to 50°C.

Using a high speed homogenator, Solution A is dispersed in Solution B. The emulsion is cooled down to room temperature.

The protease activity of the protease water-in-oil emulsion is about 17,000 U/g emulsion. The recovery of protease from both Solution A and the water-in-oil emulsion was approximately 90%, which illustrates that emulsification has no significant detrimental effects on proteolytic activity. The storage stabilities of the emulsion kept at either 25°C or 35°C are shown in Table 2.

**Table 2.**

| Storage stability of the protease emulsion preparation. | | | | | | |
|---|---|---|---|---|---|---|
| TIME (week) | PROTEOLYTIC ACTIVITY | | | | DROPLET DIAMETER µ | |
| | 25°C | | 35°C | | | |
| | U*/g emulsion | % | U*/g emulsion | % | 25°C | 35°C |
| 0 | 16,926 | 100 | 16,926 | 100 | 2 - 10 | 2 - 10 |
| 1 | 12,078 | 71 | 14,719 | 87 | 2 - 10 | 2 - 10 |
| 2 | 19,957 | 118 | 17,446 | 103 | --- | --- |
| 3 | 17,056 | 101 | 14,892 | 88 | --- | --- |
| 4 | 15,281 | 90 | 14,113 | 83 | --- | --- |
| 5 | 14,848 | 88 | 12,468 | 74 | --- | --- |
| 6 | 15,844 | 94 | 7,143 | 42 | 2 - 10 | 2 - 10 |
| 7 | 13,420 | 79 | 10,736 | 63 | --- | --- |
| 5 (month) | --- | -- | --- | -- | 2 - 15 | --- |

| | | | | | | |
|---|---|---|---|---|---|---|
| * A unit of protease activity is the amount of enzyme activity which produces an amount of hydrolysate from casein (Hammersten, Merck) at pH 7.0 and 37°C, having a similar optical density at 275 nm as a tyrosine solution of 1.5 µg/ml | | | | | | |

### Example 3

### Enzyme activity in a stable phytase water-in-oil emulsion

### Solution A:

Under gentle stirring 100 g sorbitol is dissolved in 100 g of Natuphos® UF-concentrate.

### Solution B:

Under gentle stirring 24 g of Hymono™ 1163 is dissolved in 700 g of fish oil at a temperature of 70°C. Subsequently the solution is cooled to 50°C.

Using a high speed homogenator, Solution A is dispersed in Solution B. The emulsion is cooled down to room temperature.

The recovery of phytase activity from both Solution A and the phytase water-in-oil emulsion was approximately 90%. This illustrates that emulsification has no detrimental effects on the phytic acid degrading activities of the enzyme. Phytase activity was determined as described in EP 0 420 358.

The storage stabilities of the emulsion kept at either 25°C or 35°C are shown in Table 3.

**Table 3.**

| Storage stability of the phytase emulsion preparation. | | | | | | |
|---|---|---|---|---|---|---|
| TIME (week) | PHYTASE ACTIVITY | | | | DROPLET DIAMETER µ | |
| | 25°C | | 35°C | | | |
| | U*/g emulsion | % | U*/g emulsion | % | 25°C | 35°C |
| 0 | 531 | 100 | 531 | 100 | 2 - 10 | 2 - 10 |
| 1 | 623 | 117 | 569 | 107 | 2 - 10 | 2 - 10 |
| 3 | 494 | 93 | 654 | 123 | --- | --- |
| 4 | 616 | 116 | 528 | 99 | --- | --- |
| 5 | 523 | 98 | 337 | 63 | --- | --- |
| 6 | 513 | 97 | 451 | 85 | 2 - 10 | 2 - 15 |
| 7 | 295 | 56 | 380 | 71 | --- | --- |
| 5 (month) | --- | -- | --- | -- | 2 - 15 | --- |

| | | | | | | |
|---|---|---|---|---|---|---|
| * A unit of phytase activity is defined as that amount of enzyme which liberates inorganic phosphorus from 1.5 mM sodium phytate at the rate of 1 µmol/min at 37°C and at a pH of 5.50 | | | | | | |

### Example 4

### Preparation of stable phytase and protease water-in-oil emulsions using polyglycerol polyricinoleate

### 4a. Phytase/oil emulsion

Either 3.0 g or 9.0 g Admul™ W 1403 was dissolved in 87.5 g fish oil, whereupon an emulsion was prepared with 75.0 g Natuphos® 5000 containing 50% sorbitol, using an Ultra Turrax during 2* 30 seconds at full speed. The Natuphos® which was stabilized with 40% sorbitol has been brought to 50% using additional sorbitol. As a result, the calculated activity will be 4100 U/g instead of 5000 U/g.

### The breaking of the emulsion:

The emulsion is slowly deep frozen until -20°C and is kept at this temperature during 20 hours. Subsequently, it is brought to room temperature and centrifuged at 20,000 rpm in a Sorvall centrifuge (SM 24 rotor) for 30 minutes. During this treatment the temperature is 20-25°C.

The emulsion containing 3.0 g of emulsifier is now separated in 3 layers, i.e. a clear oil layer, a thin interphase and a clear water layer. In this water layer, phytase activity has been determined to be 3750 U/g, demonstrating that phytase activity has been recovered for at least 90%.

The emulsion containing 9.0 g of emulsifier could not be separated by centrifugation. Kept at room temperature, the latter emulsion remained physically stable for a period of 6 months at least. This observation is in accordance with the superior stability of the phytase emulsion described in Example 1.

### 4b. Protease/oil emulsion

A mixture of 87.5 g fish oil, 3.0g Admul™ WOL 1403 (dissolved in fish oil) and 75.0 g Protease B500 in a solution of 50% sorbitol was homogenized in the same way as described above for the phytase/oil emulsion. Quite surprisingly, the combination of this proteolytic enzyme with the Admul™ WOL emulsifier generated emulsions which exhibited a decreased stability as compared to the stability of Protease B500 in combination with the Hymono™ emulsifier (see Example 2). Obviously, the Hymono™ emulsifier is to be preferred in preparing the protease B500 emulsions.

### Example 5

### Stability of phytase in emulsions containing proteases

To demonstrate the protecting effect of the emulsion on incompatible compounds, water-in-oil emulsions containing both phytase and an acid protease were made. This example demonstrates that a mixture of emulsions containing either the phytase or the acid protease in an aqueous phase is more stable than an emulsion containing the two enzymes mixed in one aqueous phase.

### Materials

Natuphos® UF concentrate (stabilized with 50% sorbitol by diluting a 70% sorbitol solution obtained from Roquette Frères, France).
Fromase® 150 TL (a liquid acid protease from Mucor miehei as obtained from Gist-brocades).
Admul™ WOL 1403 (from Quest, Holland).
Fish oil (capelin oil from Skretting, Norway)

### 5a. Natuphos® /oil emulsion

Following the protocol outlined in Example 4, a stable emulsion was obtained by dissolving 6 g Admul™ WOL in 87.5 g fish oil and subsequent vigorous mixing of the oil with 75 g Natuphos® UF concentrate. The emulsion was kept at 20°C. A first sample was immediately frozen at -20°C.

### 5b. Fromase® /oil emulsion

Two ml of Fromase® 150 TL were added to 98 ml water containing 50% sorbitol. The standard emulsion was prepared by mixing 87.5 g fish oil, 6 g Admul™ WOL and 75 g of the Fromase® solution. The emulsion was kept at 20°C.

### 5c. The mixed emulsion

Immediately after preparation of emulsions (5a) and (5b), a mixed emulsion was prepared by adding 100 ml of the Natuphos® /oil emulsion to 100 ml of the Fromase® /oil emulsion. After thorough handmixing, the mixture was stored at 20°C. A first sample was immediately frozen at -20°C.

### 5d. Natuphos® -Fromase® /oil emulsion

To 75 g of the Natuphos® UF concentrate with sorbitol 750 µl of Fromase® TL was added and then mixed. Addition of 87.5 g fish oil containing 6 g Admul™ WOL followed by vigorous mixing yielded the desired emulsion. The emulsion was stored at 20°C. A first sample was immediately frozen at -20°C.

### 5e. Stability of phytase in the various emulsions

Kept at 20°C, the various emulsions were sampled after 5 and 10 days of storage and immediately frozen at -20°C. After at least one night at -20°C, the various samples were thawed and centrifuged as described in Example 4. Phytase activities were determined by samples taken from the aqueous layer. Assuming 100% activity in the various samples obtained from the Natuphos® /oil emulsion (5a), the following phytase activities were recorded after 5 respectively 10 days of storage.
In the mixed emulsion (5c): 94% respectively 96% of the initial phytase activity.
In the Natuphos® -Fromase® oil emulsion (5d): 80% respectively 78% of the initial phytase activity.

Taking into account that the proteolytic activity of the Fromase® is relatively low due to the presence of the sorbitol, the data illustrate that the emulsion adds to the stability of formulated labile compounds. Phytase is protected against proteolytic attack when separately emulsified from the protease.

## Claims

1. A water-in-oil emulsion comprising water, oil, an enzyme, a water activity-lowering polyol and an emulsifier, **characterized in that** the enzyme and the water activity-lowering polyol are present in the water phase of the emulsion, and the water activity-lowering polyol is present in the water phase in a concentration of 30-70% (w/w).

2. The emulsion according to claim 1, **characterized in that** the polyol is selected from the group comprising glycerol, sorbitol, sucrose, poly propylene glycol, lactose, trehalose, maltodextrins and glucose.

3. The emulsion according to claim 1 or 2, **characterized in that** the emulsifier is selected from the group of emulsifiers which give rise to a water-in-oil emulsion.

4. The emulsion according to any one of claims 1 to 3, **characterized in that** the emulsifier is active in the presence of high concentrations of the polyol.

5. The emulsion according to claim 3 or 4, **characterized in that** the emulsifier is selected from the group of monoglycerides and polyglycerol polyricinoleate.

6. The emulsion according to any one of claims 1 to 5, **characterized in that** the enzyme is selected from the group comprising proteases, carbohydrases, lipases, phospholipases, oxidoreductases and phytases.

7. Use of an emulsion according to any one of claims 1 to 6 in the preparation of a human or animal food or cosmetic.

8. A human or animal food or a cosmetic comprising an emulsion according to any one of claims 1 to 6.

9. A method for treating a food or feed particle **characterized int hat** an emulsion according to any one of claims 1 to 6 is used to spray the particles.

## Patentansprüche

1. Wasser-in-Öl-Emulsion, die Wasser, ein Öl, ein Enzym, ein die Wasseraktivität verminderndes Polyol und einen Emulgator enthält, **dadurch gekennzeichnet, daß** das Enzym und das die Wasseraktivität vermindernde Polyol in der Wasserphase der Emulsion vorliegen und das die Wasseraktivität vermindernde Polyol in der Wasserphase in einer Konzentration von 30-70 % (Gew./Gew.) enthalten ist.

2. Emulsion nach Anspruch 1, **dadurch gekennzeichnet, daß** das Polyol aus Glycerin, Sorbit, Saccharose, Polypropylenglykol, Lactose, Trehalose, Maltodextrinen und Glucose ausgewählt ist.

3. Emulsion nach Anspruch 1 oder 2, **dadurch gekennzeichnet, daß** der Emulgator aus der Gruppe von Emulgatoren ausgewählt ist, die zu einer Wasser-in-Öl-Emulsion führen.

4. Emulsion nach einem der Ansprüche 1 bis 3, **dadurch gekennzeichnet, daß** der Emulgator in Gegenwart hoher Polyolkonzentrationen aktiv ist.

5. Emulsion nach Anspruch 3 oder 4, **dadurch gekennzeichnet, daß** der Emulgator aus der Gruppe der Monoglyceride und Polyglycerinpolyricinoleate ausgewählt ist.

6. Emulsion nach einem der Ansprüche 1 bis 5, **dadurch gekennzeichnet, daß** das Enzym aus Proteasen, Carbohydrasen, Lipasen, Phospholipasen, Oxoreduktasen und Phytasen ausgewählt ist.

7. Verwendung einer Emulsion nach einem der Ansprüche 1 bis 6 bei der Herstellung eines Human- oder Tiernahrungsmittels oder eines Kosmetikums.

8. Human- oder Tiernahrungsmittel oder Kosmetikum mit einem Gehalt an einer Emulsion nach einem der Ansprüche 1 bis 6.

9. Verfahren zum Behandeln eines Nahrungsmittel- oder Futtermittelteilchens, **dadurch gekennzeichnet, daß** eine Emulsion nach einem der Ansprüche 1 bis 6 zum Besprühen der Teilchen verwendet wird.

## Revendications

1. Emulsion huile dans l'eau comprenant de l'eau, de l'huile, une enzyme, un polyol abaissant l'activité dans l'eau et un émulsionnant, **caractérisée en ce que** l'enzyme et le polyol abaissant l'activité dans l'eau sont présents dans la phase aqueuse de l'émulsion, et le polyol abaissant l'activité dans l'eau est présent dans la phase aqueuse à une concentration de 30 à 70 % en poids.

2. Emulsion selon la revendication 1, **caractérisé en ce que** le polyol est choisi dans l'ensemble comprenant le glycérol, le sorbitol, le saccharose, le polypropylèneglycol, le lactose, le tréhalose, les maltodextrines et le glucose.

3. Emulsion selon la revendication 1 ou 2, **caractérisée en ce que** l'émulsionnant est choisi dans l'ensemble d'émulsionnants qui conduisent à une émulsion huile dans l'eau.

4. Emulsion selon l'une quelconque des revendications 1 à 3, **caractérisée en ce que** l'émulsionnant est actif en présence de concentrations élevées du polyol.

5. Emulsion selon la revendication 3 ou 4, **caractérisée en ce que** l'émulsionnant est choisi dans l'ensemble des monoglycérides et du polyricinoléate de polyglycérol.

6. Emulsion selon l'une quelconque des revendications 1 à 5, **caractérisé en ce que** l'enzyme est choisie dans l'ensemble comprenant les protéases, les carbohydrases, les lipases, les phospholipases, les oxydoréductases et les phytases.

7. Utilisation d'une émulsion selon l'une quelconque des revendications 1 à 6, dans la préparation d'un aliment ou cosmétique à usage humain ou animal.

8. Aliment ou cosmétique à usage humain ou animal, comprenant une émulsion selon l'une quelconque des revendications 1 à 6.

9. Procédé pour traiter une particule alimentaire à usage humain ou animal, **caractérisé en ce qu'**on utilise une émulsion selon l'une quelconque des revendications 1 à 6 pour pulvériser les particules.
